# EUROPEAN PATENT SPECIFICATION

(11) **EP 2 211 858 B1**
(45) Date of publication and mention of the grant of the patent: **25.03.2015**
(21) Application number: 08805180.0
(22) Date of filing: 09.10.2008
(51) Int. Cl.: A61K 31/4425, A61K 45/06, A61P 35/00, A61P 35/04

(54) **USE OF QUATERNARY PYRIDINIUM SALTS FOR INHIBITING CANCER METASTASES**
VERWENDUNG VON QUARTERNÄREN PYRIDINIUMSALZEN ZUR INHIBITION VON TUMOR METASTASEN
UTILISATION DE SELS DE PYRIDINIUM QUATERNAIRE POUR INHIBER DES MÉTASTASES CANCÉREUSES

(30) Priority: 12.10.2007 EP 07465008; 12.10.2007 US 998851 P
(43) Date of publication of application: 04.08.2010
(73) Proprietor: Politechnika Lodzka, 90-924 Lódz (PL); Uniwersytet Jagiellonski, 31007 Krakow (PL)
(72) Inventor: GEBICKI, Jerzy, PL-94-217 Lódz (PL); MARCINEK, Andrzej, PL-91-496 Lódz (PL); CHLOPICKI, Stefan, PL-30-047 Kraków (PL)
(74) Representative: Sitkowska, Jadwiga
(86) International application number: PCT/EP2008/063535
(87) International publication number: WO 2009/047291

(56) References cited:
- WO-A-2005/067927
- WO-A-2007/103540
- WO-A1-2006/024545
- HIRAKAWA, NOBUHIRO ET AL: "Anti-invasive activity of niacin and trigonelline against cancer cells" BIOSCIENCE, BIOTECHNOLOGY, AND BIOCHEMISTRY , 69(3), 653-658 CODEN: BBBIEJ; ISSN: 0916-8451, 2005, XP008108797
- WIECZORKOWSKA MARZENA ET AL: "Cytotoxic activity of the selected pyridinium salts against murine leukemia L1210" PHARMACOLOGICAL REPORTS, POLSKA AKADEMIA NAUK, INSTYTUT FARMAKOLOGII, KRAKOW, PL, vol. 59, no. 2, 1 January 2007 (2007-01-01), pages 216-223, XP008100966 ISSN: 1734-1140
- DATABASE EMBASE [Online] ELSEVIER SCIENCE PUBLISHERS, AMSTERDAM, NL; 1996, KOVARIK J ET AL: "Palliative radiotherapy with carbogen and nictonamide (ARCON) in the treatment of lung metastases" XP002539084 Database accession no. EMB-1996113165 & TUMOR DIAGNOSTIK UND THERAPIE 1996 DE, vol. 17, no. 1, 1996, pages 14-17, ISSN: 0722-219X
- GEBICKI J ET AL: "PRELIMINARY COMMUNICATION 1-METHYLNICOTINAMIDE: A POTENT ANTI-INFLAMMATORY AGENT OF VITAMIN ORIGIN" POLISH JOURNAL OF PHARMACOLOGY, INSTITUTE OF PHARMACOLOGY, KRAKOW, PL, vol. 55, no. 1, 1 January 2003 (2003-01-01), pages 109-112, XP008048213 ISSN: 1230-6002
- OGATA, SHIN ET AL: "Apoptosis induced by nicotinamide-related compounds and quinolinic acid in HL-60 cells" BIOSCIENCE, BIOTECHNOLOGY, AND BIOCHEMISTRY , 64(2), 327-332 CODEN: BBBIEJ; ISSN: 0916-8451, 2000, XP008108805
- KUYKENDALL JIM R ET AL: "1-Methylnicotinamide stimulates cell growth and inhibits hemoglobin synthesis in differentiating murine erythroleukemia cells." TOXICOLOGY IN VITRO : AN INTERNATIONAL JOURNAL PUBLISHED IN ASSOCIATION WITH BIBRA DEC 2007, vol. 21, no. 8, July 2007 (2007-07), pages 1656-1662, XP008108815 ISSN: 0887-2333
- TAGUCHI, HIROSHI ET AL: "Growth inhibition of cultured animal cells with nicotinic acid related compounds" BULLETIN OF THE FACULTY OF BIORESOURCES, MIE UNIVERSITY , 8, 51-7 CODEN: BFBUEF; ISSN: 0915-0471, 1992, XP008108806
- YAGASAKI, KAZUMI ET AL: "Bioavailability and inhibitory actions of trigonelline, chlorogenic acid and related compounds against hepatoma cell invasion in culture and their modes of actions" ANIMAL CELL TECHNOLOGY: BASIC & APPLIED ASPECTS, PROCEEDINGS OF THE ANNUAL MEETING OF THE JAPANESE ASSOCIATION FOR ANIMAL CELL TECHNOLOGY, 13TH, FUKUOKA AND KARATSU, JAPAN, NOV. 16-21, 2000 , MEETING DATE 2000, 421-425. EDITOR(S): SHIRAHATA, SANETAK, 2002, XP008108812

## Description

### The field of the invention

The present invention relates to compounds for use according to formula I of claim 1 for use in inhibiting and/or preventing formation of cancer metastases and secondary malignant cancers.

### Background art

The most deadly aspect of cancer is its ability to spread, or metastasize. Cancer cells initially group together to form a primary tumor. Once the tumor is formed, cells may begin to break off from this tumor and travel to other, distant parts or organs of the body. These cancer cells that travel through the body are capable of establishing new tumors in locations remote from the site of the original disease. This process is called metastasis.

The body has many self-defenses against primary cancer cells. Tumours when diagnosed may be treated and cured by one of the established methods of cancer treatment, such as surgery, i.e. removing a cancerous tissue, radiotherapy, or chemotherapy. Localized tumors that have not had the opportunity or time to metastasize have the best prognosis for cure. Cancers which have metastasized usually indicate a later stage disease, and treatment becomes more complicated, with poorer outcomes. In late stages, patients with oral cancer for example, may succumb to a cancer in the lungs or the brain, which was not the location of the original, primary tumor. Non-symptomatic and/or non-diagnosed and therefore not treated primary cancer may also spread to distant organs of the body, substantially diminishing the prognosis for later cure of both the primary and metastatic malignant cancer.

Metastasis most commonly occurs by way of the bloodstream or the lymphatic system. Just like normal cells, cancer cells must have a blood supply in order to function. They have the access to the bloodstream just as healthy cells do. This access allows detached malignant cells from the tumor to enter the bodies' general bloodstream. Once in the bloodstream, the cancer cells now have access to every portion of the body. The lymphatic system has its own channels throughout the body like the circulatory system, through which a malignant cell can travel. When surgeons remove a tumor, they may also remove nearby portions of the lymph system including the lymph nodes, as these are frequently the first sites of the cancers' metastasis. Once metastasis to the lymphatic system has occurred, the prognosis for cure drops significantly. To kill metastatic cells, patient may be offered further treatment with chemotherapy, radiotherapy or hormone therapy, i.e. so called 'adjuvant treatment'. Such adjuvant therapy involves serious inconveniencies and side effects for patients.

It is known that in order to spread, cancer cells must first detach from the primary tumour and to get into the blood stream or the circulating lymph. When they are in the blood stream or the circulating lymph, they must survive in a hostile environment, being killed by the defense forces of the body, like white blood cells, by the fast flowing blood, etc. They need to re-attach quite quickly in another part or organ of the body in order to survive and proliferate to form secondary tumour. Therefore, the agent which would be able to prevent re-attachment of spread cancer cells would offer a possibility to prevent the most deadly secondary tumour formation.

In WO00/40559 therapeutic and cosmetic uses of certain nicotinamide derivatives, 1,3-disubstituted pyridinium salts, including 1-methylnicotinamide (MNA) salts, were disclosed. It was reported that said derivatives have the utility in topical treatment of skin diseases, in particular crural ulceration, acne, psoriasis, atopic dermatitis, vitiligo, as well as burns and scalds and in wound healing. Said derivatives have also the activity of promoting hair re-growth, therefore they are useful in the treatment of hair loss of different origin.

In WO2005/067927 there is disclosed a vasoprotective activity of certain quaternary pyridinium salts, including 1-methylnicotinamide (MNA) and 1-methyl-3-acetyl-pyridinium salts.

Effects of 1-methylnicotinamide chloride (MNA) in some skin diseases were described in a recent publication (G 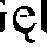 ebicki J, Sysa-J 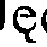 drzejowska A, Adamus J, Wozniacka A, Rybak M, Zielonka J. 1-Methylnicotinamide: a potent anti-inflammatory agent of vitamin origin. Pol. J. Pharmacol. 2003;55:109-1.12). It has been proposed that MNA displays anti-inflammatory action, though the mechanism of this effect was not elucidated.

In the publication in Pharmacological Reports, 2007, 59, 216-223, studies of cytotoxic activity of selected pyridinium salts: 1-methylnicotinamide chloride, 1-methyl-3-acetylpyridinium chloride and 1-methyl-3-nitropyridinium chloride against murine leukemia L1210 were reported by the present inventors. It was found that the cytotoxicity in cultured leukemia cells varied strongly depending on the compound used and that 3-nitro-1-methylpyridinium chloride showed the highest cytotoxicity, while cytotoxicity of 1-methylnicotinamide chloride was about 7000 lower and was observed only at very high concentration, thus being in fact negligible.

1-Methyl-3-acetylpyridinium salt (MAP), was described in a publication Takashi Sakurai, Haruo Hosoya. Charge transfer complexes of nicotinamide-adenine dinucleotide analogs and flavine mononucleotide. Biochim. Biophys. Acta 1966;112(3):359-468.

It is increasingly clear that the chemotherapy with cytotoxic agents very frequently cannot prevent metastasis. Thus, novel treatment modalities are mandatory for more efficient cancer therapy that could prevent metastasis formation while being not cytotoxic *per se.*

There remains a need for a means for inhibiting and/or preventing formation of cancer metastases and secondary malignant cancers both in non-symptomatic (apparently healthy) individuals and in individuals with detected cancer or undergoing currently the therapy of a cancer.

### Summary of the Invention

It has been now found by the present inventors that the formation of difficult to cure cancer metastases and secondary malignant cancers from spread primary cancer cells can be inhibited by the administration of an effective dose of certain selected quaternary pyridinium salt according to formula I of claim 1.

Accordingly, the invention provides a quaternary pyridinium salt of formula (I) 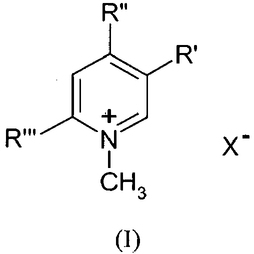 wherein R' is H; R" is selected from the group consisting of H and CH₃; R"' is selected from the group consisting of H and CH₃; and X⁻ is a pharmaceutically acceptable counterion, for use in inhibiting and/or preventing formation of cancer/tumor metastases and secondary malignant cancer/tumor.

### Brief Description of the Drawings

Fig. 1 illustrates the effect of 1,4-dimethylpyridinium (DMP) at 100 mg/kg of body weight per day on the number of lung cancer LLC cells colonies in comparison with control.
Fig. 2 illustrates the effect of 1,4-dimethylpyridinium (DMP) at 100 mg/kg of body weight per day on survival of mice after i.v. injection of cancer LLC cells in comparison with control.

### Detailed disclosure of the invention

The present invention provides a quaternary pyridinium salt of formula (I) 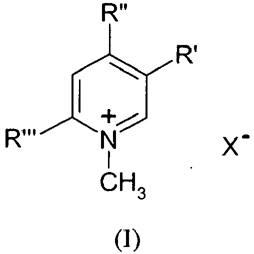 wherein R' is H; R" is selected from the group consisting of H and CH₃; R"' is selected from the group consisting of H and CH₃; and X⁻ is a pharmaceutically acceptable counterion, for use in inhibiting and/or preventing formation of cancer/tumor metastases and secondary malignant cancer/tumor.

Said inhibiting and/or preventing comprises administration to a subject of an effective amount of a quaternary pyridinium salt of formula (I)

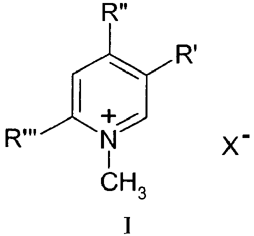

wherein R' is selected from the group consisting of H; R" is selected from the group consisting of H and CH₃; R"' is selected from the group consisting of H and CH₃; and X⁻ is a pharmaceutically acceptable counterion.

In one embodiment the compound of formula (I) is administered alone.

The compound of formula (I) can be administered alone as a preventive measure to a subject suspected to be prone to a cancer, for example on the basis of genetic implications or the previous history of family diseases.

The compound of formula (I) can be administered alone as a preventive measure to a subject which has been earlier subjected to a therapeutic treatment of a diagnosed cancer or tumour, such as chemotherapy treatment, radiotherapy treatment or a surgical treatment.

In another embodiment the compound of formula (I) is used in combination with another therapeutic treatment of a diagnosed cancer or tumour, such as chemotherapy treatment, radiotherapy treatment or a surgical treatment.

Chemotherapy treatment can be performed by any of the chemotherapy treatment regimes know from the art, depending on the type of the cancer, using conventional chemotheraputic agents known from the art for the treatment of neoplastic diseases, for example in accordance of the teaching in Goodman and Gilman's The pharmacological Basis of Therapeutics 911 th edition, McGraw-Hill, editors Laurence L Brunton, John S Lazo, Keith L Parker, Chapter 51, Antineoplastic Agents.

The following chemotheraputic agents can be mentioned by way of non-restrictive example:
alkylating agents, including nitrogen mustards such as for example cyclophosphamide and ifosfamide, ethyleneamines and methylmelamines, such as for example thiotepa, methylhydrazine derivatives such as for example procarbazine, alkylsulfonates, such as for example busulfan, nitrosoureas, such as for example carmustine, triazenes, such as for example dacarbazine and temozolomide, platinum coordination complexes, such as for example cisplatin, carboplatin and oxaliplatin;
antimetabolites, including folic acid analogs, such as for example methotrexate, pyrimidine analogs, such as for example fluorouracil, cytarabine, gemcitabine and capecitabine, purine analogs such as for example mercaptopurine, pentostatin, cladribine, fludarabine;
vinca alkaloids, such as for example vinblastine, vinorelbine and vincristine;
taxanes, such as for example paclitaxel and docetaxel;
epipodophyllotoxins, such as for example etoposide and teniposide;
camptothecins, such as for example topotecan and irinotecan;
anticancer antibiotics, such as for example dactinomycin, daunorubicin and doxorubicin;
anthracenediones, such as for example mitoxantrone, mitomycin and bleomycin;
enzymes, such as for example L-asparaginase;
substituted ureas, such as for example hydroxyurea;
differentiating agents, such as for example tretinoin;
proteine kinase inhibitors, such as for example imatinib;
proteasome inhibitors, such as for example geftinib and bortezomib;
biological response modifiers, such as for example interferon-alfa, inteleukin alfa;
antibodies;
hormones and antagonists, including adrenocortical suppresants, such as for example aminoglutethimide; adrenocorticosteroids, such as for example prednisone; progestins, such as for example megestrol acetate, medroxyprogesterone; estrogens, such as for example diethylstilbestrol; anti-estrogens, such as for example tamoxifen and toremifene; aromatase inhibitors, such as for example anastrozole, letrozole and exemestane; androgens, such as for example testosterone propionate; anti-androgens, such as for example flutamide; and gonadotropin-releasing agents, such as for example leuprolide.

A surgical treatment to be used in the combined treatment in combination with administration of the compounds of formula (I) will consist in removing a tumor together with surrounding tissue, and optionally lymph nodes, such as sentinel lymph nodes, using conventional methods known from the prior art.

A radiotherapy treatment to be used in the combined treatment in combination with administration of the compounds of formula (I) will consist in irradiation of the tumor, using conventional means and methods known from the prior art.

In the embodiment of combined treatment with another therapeutic treatment of a diagnosed cancer or tumour, such as chemotherapy treatment, radiotherapy treatment or a surgical treatment, the administration of the compounds of formula (I) can be performed before or after such another treatment, for a sufficient period. Furthermore, in the case of combined treatment with chemotherapy, a compound of the formula (I) can be administered before, during and/or after the course of a chemotherapeutic treatment regime.

It has been shown that the compounds of the above formula (I) significantly reduce the number of cancer metastases after injection of cancer cells into blood system. They also prolong survival after i.v. administration of cancer cells. It can be therefore expected that administration of the compounds can exert protective effects against cancer spread and metastases without the need of chemotherapy or as an adjunct treatment in combination with chemotherapy.

Preferred manner of administration is oral administration, in a single dose or divided dose.

Particular compounds of formula (I) useful in the present invention are selected from 1,2-dimethylpyridinium, 1,4-dimethylpyridinium, and 1,2,4-trimethylpyridinium salts.

As indicated above, in the compounds of formula (I) X⁻ can be any pharmaceutically acceptable and physiologically suitable counter-anion. Said counterion is of a non-complex type, i.e. does not contain complexed metal cations. The quaternary pyridinium salts used in the method of the present invention can thus be derived from any physiologically suitable acceptable organic or inorganic acids. Suitable inorganic acid salts include, for example, chloride, bromide, iodide and carbonate. Suitable organic acid salts include for example mono-, di-and tri-carboxylic acid salts such as acetate, benzoate, salicylate, glycolate, lactate, maleate and citrate.

Preferred salts include chloride, benzoate, salicylate, acetate, citrate and lactate. Especially preferred are chloride salts, due to physiological acceptability of the chloride anion.

Without being bound by any theoretical considerations, the inventors believe that the compounds of the above formula (I), while being not cytotoxic *per se,* can probably act by preventing primary cancer cells present in the blood or lymph system from re-attachment to the tissue of another organ or part of the body.

In one embodiment the compounds of the above formula (1) can be particularly useful to prevent micrometastases. i.e. metastases that are too small to be seen.

It is in the skills of the ordinary practitioner in the field of cancer treatment to determine whether a patient is likely to have micrometastases in the future. Without limitation, the patient is likely to have micrometastasis if for example cancer cells are found in the blood vessels in the tumour tissue removed during surgery, the grade of the primary cancer is very high, or lymph nodes that were removed at operation contained cancer cells.

The additional advantage of the compounds of formula (I), besides their ability to prevent formation of metastases, can be expected in the case of administration thereof to patients who receive or who received earlier chemotherapeutic treatment with cytotoxic agents. Due to their vasoprotective effect, the compounds of formula (I) could exert healing and protective effect on the cardiovascular system injuries and impairments caused by anti-cancer chemotherapeutics, such as for example anthracycline chemotherapeutics.

These and other embodiments of the invention will be described with reference to following definitions.

The terms "chemotherapy", "radiotherapy" and "surgery" are used herein in accordance with their established meanings in the art.

The term "subject" includes living organisms in which the treatment is performed. The term "subject" includes animals (e.g., mammals, e.g., cats, dogs, horses, pigs, cows, goats, sheep, rodents, e.g., mice or rats, rabbits, squirrels, bears, primates (e.g., chimpanzees, monkeys, gorillas, and humans)), as well as chickens, ducks, geese, and transgenic species thereof, and cells derived therefrom. In a preferred embodiment, the term "subject" refers to a human patient.

Said cancer can be a solid tumor or cancer.

Said tumor or cancer can be selected from the group consisting of bladder cancer, brain cancer, breast cancer, colorectal cancer, endometrial cancer, esophagus cancer, head and neck cancer, Hodgkin's disease (lymphoma), lung cancer, non-Hodgkin's lymphoma, oral cancer, ovarian cancer, prostate cancer, uterine cancer, abdominal cancer, unilateral and bilateral acoustic neuroma, pituitary tumor, pituitary tumor and non-pituitary tumor in acromegaly, pituitary tumor and non-pituitary tumor in gigantism, adenocarcinoma, arteriovenous malformation, blood cancer, cervical cancer, Cushing's syndrome, Ewing's sarcoma, fibrosarcoma, galacatorrhea, glioblastoma, Graves disease, hyperthyroidism, Kaposi's sarcoma, kidney cancer, larynx cancer, liver cancer, melanoma, meningioma, mesothelioma, multiple myeloma, mycosis fungoides, neuroblastoma, neurofibromatosis type 1 and 2, oligodendroglioma, orbit tumour, osteosarcoma (cancer of the bone), pancreatic cancer, polycythemia, retinoblastoma, rhabdomyosarcoma, rodent ulcer (basal cell carcinoma), skin cancer, stomach cancer, testicular cancer, thyroid cancer, vagina cancer, vulva cancer, Wilm's tumour, Peyronies disease, squamous cells papilloma, squamous cells carcinoma, liposarcoma, chondrosarcoma, angiosarcoma, synovial sarcoma, leiomyosarcoma, rhabdomyosarcoma, cystadenocarcinoma, bronchogenic carcinoma, and bronchial adenoma, without any limitation to the foregoing.

Brain cancers include, without limitation, primary brain tumors caused by cancer of brain cells, metastatic brain cancer (cancer of another part of the body has spread to the brain) benign brain tumor, primary CNS lymphoma and brain sarcoma.

Bladder cancers include, without limitation, transitional cell carcinoma, squamous cell carcinomas and superficial bladder cancer.

Blood cancer includes, without limitation, leukemia, lymphoma and myeloma.

Breast cancers include, without limitation, breast carcinoma, breast sarcoma, Paget's Disease, lobular carcinoma in situ and ductal carcinoma in situ.

Colorectal cancer include stage 0 to stage III colorectal cancer.

Esophagus cancer includes, without limitation, esophagus squamous cell carcinoma and esophagus adenocarcinoma.

Lung cancer includes, without limitation, primary lung cancer, including small cell lung cancer and non-small cell lung cancer, as well as metastatic lung cancer that results from metastasis of other cancers, such as breast cancer, colon cancer, rectal cancer and stomach cancer.

Melanoma includes, without limitation, cutaneous melanoma, ocular melanoma, melanoma of the meninges, melanoma of digestive tract, and melanoma of lymph nodes.

Mesothelioma includes, without limitation, pleural mesothelioma, peritoneal mesothelioma, perineal mesothelioma, malignant mesothelioma and benign mesothelioma.

Neuroblastoma includes, without limitation, adrenal neuroblastoma, neuroblastoma of the brain, abdominal neuroblastoma and chest neuroblastoma.

Oral cancer includes, without limitation, tongue cancer, pharynx cancer, lip cancer and gum cancer.

Pituitary cancer includes, without limitation, prolactinoma, pituitary adenoma, corticotropinoma, luteinizing hormone (LH) secreting tumors, follicle stimulating hormone (FSH) secreting tumors.

Skin cancer includes, without limitation, skin basal cell carcinoma (rodent ulcer), skin squamous cell carcinoma and melanoma.

Vagina cancer includes, without limitation, embryonal rhabdomyosarcomas and DES-related vaginal cancer.

Some of the compounds of formula (I) are commercially available. Alternatively, the compounds can be easily prepared from commercially available compounds (including nicotinamide and nicotinic acid) by synthetic methods well-known to a person skilled in the art. Such methods would include synthesis from appropriately substituted pyridine compounds without methyl group at the pyridine nitrogen atom, i.e. in a position 1 of the pyridine ring. Quaternary pyridinium compounds of formula (I) wherein X⁻represents halogen anion can be prepared starting from corresponding pyridine compounds by direct methylation with methyl halogenide in a manner known *per se.* Quaternary pyridinium compounds of formula (1) wherein X⁻ represents chloride anion can be prepared by direct methylation with methyl chloride, such as disclosed in AT131118, GB348345, US3614408, and US4115390. Quaternary pyridinium compounds of formula (I) where X⁻ is a non-halogen anion can be prepared by substitution of a halogen anion with another non-halogen anion, for example by the treatment with a salt of such another anion, such as for example sodium or silver salt of another anion. As an illustration, lactates and acetates of the compounds of formula (I) can be prepared by the treatment of a halogenide, preferably chloride, with silver lactate or acetate, respectively. Salicylates of the compounds of formula (I) can be prepared by the treatment of a halogenide, preferably chloride, with sodium salicylate.

In accordance with the invention the compounds of formula (I) can be administered orally or by injection, using any pharmaceutical dosage forms (pharmaceutical compositions) known for a person skilled in the art for such administration.

For oral administration both solid and liquid formulations can be used. Solid formulations include conventional tablets, capsules, troches, powders for reconstitution in liquids, such as water or juices. Any suitable conventional excipients can be used for the preparation of such solid forms. Liquid forms for oral administration include in particular aqueous solutions, with the addition of any conventional excipients, such as for example flavours and/or sweeteners.

The administration by injection includes bolus intravenous injection, continuous intravenous infusion, as well as subcutaneous injection. Any suitable injections formulations can be used, such as for example aqueous saline solutions, buffered saline solutions, etc. using conventional excipients known from the art, such as preservatives, isotonic agents, buffers.

Actual dosage levels of the compound of formula (I) in the use of this invention may be varied so as to obtain an amount of the active ingredient which is effective to achieve the desired therapeutic response for a particular patient, composition, and mode of administration, without being toxic to the patient.

Dosage regimens can be in the range 5 mg to 5 g of the compound of formula (I) in a single or divided doses per day. In particular, dosages such as 5 mg to 1 g can be used, or 5 mg to 500 mg, In some embodiments, a dose the compound of formula (I) is about 100 mg, or about 80 mg, or about 60 mg, or about 50 mg, or about 30 mg, or about 20 mg, or about 10 mg, or about 5 mg, per day.

The selected dosage level will depend upon a variety of factors including the activity of the particular compound of the present invention employed, the type of radiotherapy, the time of administration, the rate of excretion of the particular compound being employed, the duration of the treatment, other drugs, compounds or materials used in combination with the particular compound and radiotherapy employed, the age, sex, weight, condition, general health and prior medical history of the patient being treated, and like factors well known in the medical arts.

A medical doctor, e.g., physician or veterinarian, having ordinary skill in the art can readily determine and prescribe the effective amount of the pharmaceutical composition required. For example, the physician or veterinarian could start doses of the compounds of the invention at levels lower than that required in order to achieve the desired therapeutic effect and gradually increase the dosage until the desired effect is achieved.

One of ordinary skill in the art would be able to study the relevant factors and make the determination regarding the effective amount of the therapeutic compound without undue experimentation.

The invention will be further illustrated by reference to the following examples which describe in details methods for assaying biological activity of the compounds.

### Examples

The following abbreviations are used herein.
DMP - 1,4-dimethylpyridinium

### Example 1

### Effect of DMP on the number of tumour lung colonies

Six-to-eight-week old male C57BL/6 mice obtained from Medical Research Center of the Polish Academy of Sciences Warsaw, Poland were used for the experiments. To obtain the cells for the assay, 14 days after subcutaneous (s.c.) transplantation of 10⁶ Lewis Lung Cancer (LLC) cells to a mouse the developed tumor was removed, minced, and incubated for 30 min at room temperature with 0.25% trypsin-EDTA and a standard DNase 1 enzyme solution. After that, the cells were washed and resuspended in culture medium (CM: RPMI-1640 medium supplemented with 10% FBS, 100 U/ml penicillin, 100 µg/ml streptomycin, and 2 mM L-glutamine). Then, the mice were intravenously (i.v.) injected with 5×10⁵ LLC cells/mouse. Fourteen days later the animals were killed, their lungs injected with India ink, and visible colonies on the lung's surface were counted using the method described in A. Cheda, J. Wrembel-Wargocka, E. Lisiak, E. M. Nowosielska, M. Marciniak, M.K. Janiak: Single Low Doses of X-Rays Inhibit the Development of Experimental Tumor Metastases and Trigger the Activities of NK Cells in Mice. Radiat. Res. 161: 335-340, 2004, and in E.M. Nowosielska, A. Cheda, J. Wrembel-Wargocka, M.K. Janiak: Modulation of the growth of pulmonary tumor colonies in mice after single or fractionated low-level irradiations with X-rays. Nukleonika 53, Supl. 1: S9-S15, 2008.

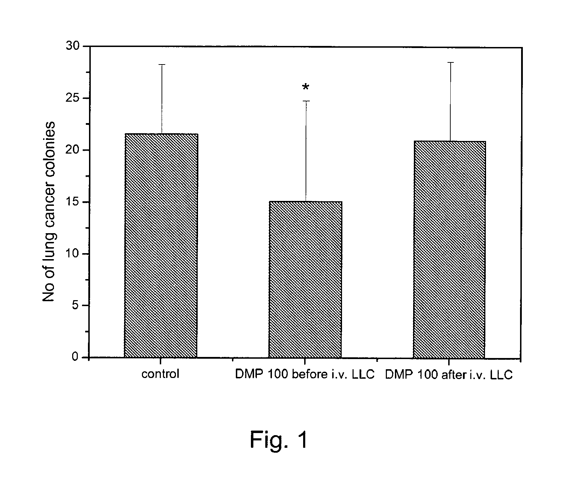

The mice were divided into control group receiving no treatment and treatment group receiving tested compound DMP as its chloride salt. The treatment group was divided into two subgroups, one receiving tested compound starting from day 7 before injection of the LLC cells and the second receiving tested compound starting on day 7 after injection of the LLC cells. The compounds was given dissolved in drinking water at 100 mg/kg of body weight/24 h. The results of the experiments are shown in Fig. 1. Statistical significance with respect to the control (Mann Whitney U test): P value < 0.05 (*); < 0.01 (**), < 0.005 (***).

It can be seen that tested compound significantly reduces the number of cancer metastases after injection of cancer cells into blood system.

### Example 2

### Effect of DMP on the survival of mice after i.v. injection of the LLC cells

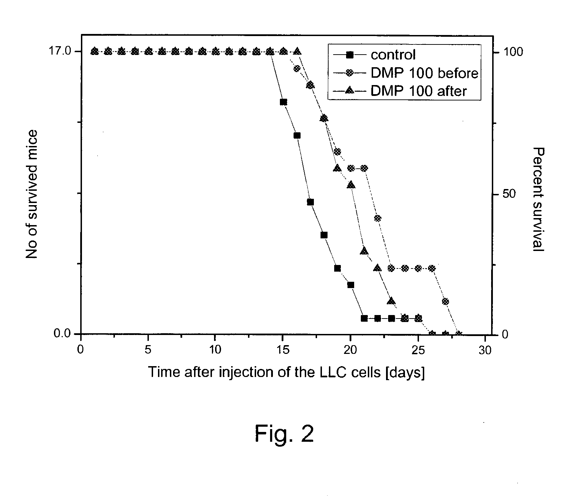

Survival of the C57BL/6 mice after i.v. injection of 5×10⁵ LLC cells/mouse was assessed. The mice were divided into control group and treatment group receiving DMP chloride at 100 mg/kg of body weight/24 h. The treatment group was further subdivided into mice receiving the tested compound starting from day 7 before injection of the LLC cells and mice receiving the tested compound starting from day 7 after injection of the LLC cells. The tested compound was given dissolved in drinking water until death of the animals. The results of the tests are presented on Fig. 2. Statistical significance with respect to the control (Mantel-Cox test): P value < 0.001 (DMP 100 before);< 0.05 (DMP 100 after).

It can be seen that tested compound prolongs survival after i.v. administration of cancer cells.

## Claims

1. A compound of formula (I) 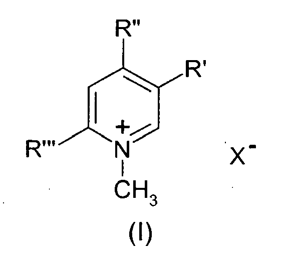 wherein R' is H; R" is selected from the group consisting of H and CH₃; R'" is selected from the group consisting of H and CH₃; and X⁻ is a pharmaceutically acceptable counterion for use in inhibiting and/or preventing formation of cancer/tumor metastases and secondary malignant cancer/tumor.

2. The compound for use of claim 1 wherein the cancer is selected from solid tumors.

3. The compound for use of claim 1 or 2 wherein the compound of formula (I) is selected from 1,2-dimethylpyridinium, 1,4-dimethylpyridinium, and 1,2,4-trimethylpyridinium salts.

4. The compound for use of any one of claims 1 to 3 wherein the compound of formula (I) is administered alone.

5. The compound for use of any one of claims 1 to 3 wherein the compound of formula (I) is administered in combination with other therapeutic cancer treatment.

6. The compound for use of claim 5 wherein said other therapeutic cancer treatment is selected from a chemotherapy, a radiotherapy, hormone therapy, immunotherapy, ablation and surgery of a cancer.

7. The compound for use of any of claims 1 to 6 wherein said tumor or cancer is selected from the group consisting of bladder cancer, brain cancer, breast cancer, colorectal cancer, endometrial cancer, esophagus cancer, head and neck cancer, Hodgkin's disease (lymphoma), lung cancer, non-Hodgkin's lymphoma, oral cancer, ovarian cancer, prostate cancer, uterine cancer, abdominal cancer, unilateral and bilateral acoustic neuroma, pituitary tumor, pituitary tumour and non-pituitary tumor in acromegaly, pituitary tumour and non-pituitary tumor in gigantism, adenocarcinoma, arteriovenous malformation, blood cancer, cervical cancer, Cushing's syndrome, Ewing's sarcoma, fibrosarcoma, galacatorrhea, glioblastoma, Graves disease, hyperthyroidism, Kaposi's sarcoma, kidney cancer, larynx cancer, liver cancer, melanoma, meningioma, mesothelioma, multiple myeloma, mycosis fungoides, neuroblastoma, neurofibromatosis type 1 and 2, oligodendroglioma, orbit tumour, osteosarcoma (cancer of the bone), pancreatic cancer, polycythemia, retinoblastoma, rhabdomyosarcoma, rodent ulcer (basal cell carcinoma), skin cancer, stomach cancer, testicular cancer, thyroid cancer, vagina cancer, vulva cancer, Wilm's tumour, Peyronies disease, squamous cells papilloma, squamous cells carcinoma, liposarcoma, chondrosarcoma, angiosarcoma, synovial sarcoma, leiomyosarcoma, rhabdomyosarcoma, cystadenocarcinoma, bronchogenic carcinoma, and bronchial adenoma.

8. The compound for use of any of claims 1 to 7 wherein said tumor or cancer is selected from the group consisting of breast cancer, lung cancer, colon and rectum cancer, ovary cancer, prostate cancer and urinary tract cancer.

9. The compound for use of any of claims 1 to 8 wherein the compound of formula (I) is administered orally.

## Patentansprüche

1. Die Verbindung der Formel (I) 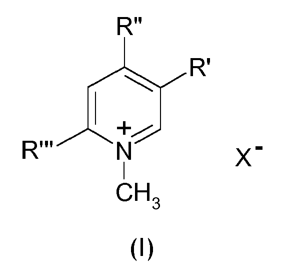 worin R' für H steht, R" aus der Gruppe bestehend aus H und CH₃ ausgewählt ist, R'" aus der Gruppe bestehend aus H und CH₃ ausgewählt ist, und X⁻ für ein pharmazeutisch annehmbares Gegenion steht, zur Verwendung in der Hemmung und/oder Prophylaxe der Bildung von Krebs/Tumormetastasen und sekundären bösartigen Krebs/Tumoren.

2. Verbindung zur Verwendung nach Anspruch 1, wobei der Krebs aus festen Tumoren ausgewählt ist.

3. Verbindung zur Verwendung nach einem der Ansprüche 1 oder 2, wobei die Verbindung der Formel (I) aus 1,2-Dimethylpyridinium, 1,4-Dimethylpyridinium und 1,2,4-Trimethylpyridinium Salze ausgewählt ist.

4. Verbindung zur Verwendung nach einem der Ansprüche 1 bis 3, wobei die Verbindung der Formel (I) allein verabreicht wird.

5. Verbindung zur Verwendung nach einem der Ansprüche 1 bis 3, wobei die Verbindung der Formel (I) in Kombination mit anderen therapeutischen Behandlung von Krebs verabreicht wird.

6. Verbindung zur Verwendung nach Anspruch 5, wobei das andere therapeutische Behandlung von Krebs aus einer Chemotherapie, Strahlentherapie, Hormontherapie, Immuntherapie, Ablation und Operation eines Krebses ausgewählt ist.

7. Verbindung zur Verwendung nach einem der Ansprüche 1 bis 6, wobei das Tumor oder Krebs aus der Gruppe bestehend aus Blasenkrebs, Gehirnkrebs, Brustkrebs, Dickdarmkrebs, Gebärmutterkrebs, Speiseröhrenkrebs, Kopf- und Halskrebs, Hodgkin-Krankheit (Lymphom), Lungenkrebs, Non-Hodgkin-Lymphom, Mundkrebs, Eierstockkrebs, Prostatakrebs, Gebärmutterkrebs, Unterleibskrebs, uni- und bilaterale Akustikusneurinom, Hypophysentumor, Hypophysentumor und nicht-Hypophysentumor in Akromegalie, Hypophysentumor und Nicht-HypophysenTumor in Gigantismus, Adenokarzinom, arteriovenöse Malformation, Blutkrebs, Gebärmutterhalskrebs, Cushing-Syndrom, Ewing-Sarkom, Fibrosarkom, Galaktorrhoe, Glioblastom, Basedow-Krankheit, Hyperthyreose, Kaposi-Sarkom, Nierenkrebs, Kehlkopfkrebs, Leberkrebs, Melanom, Meningiom, Mesotheliom, Multiples Myelom, Mycosis fungoides, Neuroblastom, Neurofibromatose Typ 1 und 2, Oligodendrogliom, Umlaufbahn Tumor, Osteosarkom (Knochenkrebs), Pankreaskarzinom, Polyzythämie, Retinoblastom, Rhabdomyosarkom, Nagetiergeschwür (Basaliom), Hautkrebs, Magenkrebs, Hodenkrebs, Schilddrüsenkrebs, Vagina Krebs Vulva Krebs, Wilms-Tumor, Peyronie-Krankheit, Plattenepithel-Zellen Papillom, Plattenepithel-Zellen-Karzinom, Liposarkom, Chondrosarkom, Angiosarkom, Synovialsarkom, Leiomyosarkom, Rhabdomyosarkom, Zystadenokarzinom, Bronchialkarzinom und bronchiale Adenom, ausgewählt ist.

8. Verbindung zur Verwendung nach einem der Ansprüche 1 bis 7, wobei das Tumor oder Krebs aus der Gruppe bestehend aus Brustkrebs, Lungenkrebs, Dickdarm- und Mastdarmkrebs, Eierstockkrebs, Prostatakrebs und Harnwege Krebs, ausgewählt ist.

9. Verbindung zur Verwendung nach einem der Ansprüche 1 bis 8, wobei die Verbindung oral verabreicht wird.

## Revendications

1. Le compose de la formule (I) 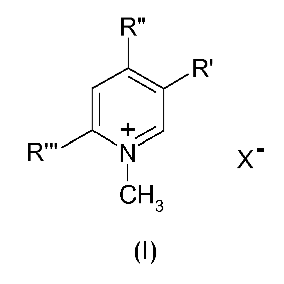 dans laquelle R' est H, R" est choisi dans le groupe consistant en H et CH₃, R"' est est choisi dans le groupe consistant en H et CH₃, et X⁻ est un contreion pharmaceutiquement acceptable, pour utilisation dans l'inhibition et/ou prévention la formation de métastases de cancer/tumeur et secondaire cancer/tumeur.

2. Le compose pour utilisation selon la revendication 1, dans lequel le cancer est choisi parmi des tumeurs solides.

3. Le compose pour utilisation selon la revendication 1 ou 2, dans lequel le composé est choisi parmi les sels de 1,2-dimethylpyridinium, 1,4-dimethylpyridinium, et 1,2,4-trimethylpyridinium.

4. Le compose pour utilisation selon l'une quelconque des revendications 1 à 3, dans lequel le composé est administré seul.

5. Le compose pour utilisation selon l'une quelconque des revendications 1 à 3, dans lequel le composé est administré en combinaison avec d'autre traitement thérapeutique du cancer.

6. Le compose pour utilisation selon la revendication 5, dans lequel ledit autre traitement thérapeutique du cancer est choisi parmi une chimiothérapie, une radiothérapie, une hormonothérapie, une immunothérapie, une intervention chirurgicale et l'ablation du cancer.

7. Le compose pour utilisation selon l'une quelconque des revendications 1 à 6, dans lequel la tumeur ou le cancer est choisi dans le groupe consistant en le cancer de la vessie, le cancer du cerveau, le cancer du sein, le cancer colorectal, le cancer de l'endomètre, le cancer de l'oesophage, de la tête et du cou, de la maladie de Hodgkin (lymphome), le cancer du poumon, un lymphome non hodgkinien, le cancer de la bouche, le cancer de l'ovaire, le cancer de la prostate, le cancer de l'utérus, le cancer abdominal, le névrome acoustique unilatérale et bilatérale, la tumeur de l'hypophyse, de l'hypophyse tumeur et tumeur non hypophysaire dans l'acromégalie, de l'hypophyse tumeur et non-hypophyso tumeur dans le gigantisme, l'adénocarcinome, la malformation artério-veineuse, le cancer du sang, le cancer cervical, le syndrome de Cushing, le sarcome, le fibrosarcome, la galactorrhée, le glioblastome, la maladie de Graves, l'hyperthyroïdie d'Ewing, le sarcome de Caposi, le cancer du rein, le cancer du larynx, le cancer du foie, le mélanome, le méningiome, le mésothéliome de Kaposi, myélome multiple, le mycose fongoïde, le neuroblastome, la neurofibromatose de type 1 et 2, l'oligodendrogliome, l'orbite tumeur, l'ostéosarcome (cancer des os), le cancer, la polyglobulie, le rétinoblastome, le rhabdomyosarcome, l'ulcère de rongeur (carcinome basocellulaire) du pancréas, le cancer de la peau, le cancer de l'estomac, le cancer des testicules, le cancer de la thyroïde, le cancer du vagin, le cancer de la vulve, la tumeur de Wilm, la maladie de Peyronie, le papillome des cellules squameuses, le carcinome des cellules squameuses, le liposarcome, le chondrosarcome, l'angiosarcome, le sarcome synovial, le léiomyosarcome, le rhabdomyosarcome, le cystadénocarcinome, le carcinome bronchogénique, et l'adénome bronchique.

8. Le compose pour utilisation selon la revendication 7, dans lequel la tumeur ou le cancer est choisi dans le groupe constitué par le cancer du sein, le cancer du poumon, le cancer du côlon et du rectum, le cancer de l'ovaire, le cancer de la prostate et le cancer des voies urinaires.

9. Le compose pour utilisation selon l'une quelconque des revendications 1 à 8, dans lequel ledit compose est administré par voie orale.
